# EUROPEAN PATENT APPLICATION

(11) **EP 4 538 322 A1**
(43) Date of publication of application: **16.04.2025**
(21) Application number: 22945615.7
(22) Date of filing: 14.12.2022
(51) Int. Cl.: C08L 1/04, C08L 5/12, C08J 9/26

(54) **POLYMERIC MICROPARTICLE HAVING PORE CHANNELS OF TWO SIZES AND PREPARATION METHOD THEREFOR**

(30) Priority: 07.06.2022 CN 202210632048
(71) Applicant: Smart Liquid Crystal Technologies Co., Ltd., Suzhou, Jiangsu 215500 (CN)
(72) Inventor: YANG, Min, Suzhou, Jiangsu 215500 (CN); WANG, Rui, Suzhou, Jiangsu 215500 (CN)
(74) Representative: Gille Hrabal Partnerschaftsgesellschaft mbB Patentanwälte
(86) International application number: PCT/CN2022/139032
(87) International publication number: WO 2023/236487

(57) **Abstract**

The present invention relates to a polymeric microparticle with dual size pores, and a preparation method therefor. The polymeric microparticle is formed by cross-linking at least partially cross-linkable oligomer materials, including rigid nanoparticles, wherein at least one of the rigid nanoparticles has a non-spherical shape in a solution. The polymeric microparticles have two sets of pores having distinctive sizes distributed inside, wherein the first set of pores are macropores larger in size, and the second set of pores are gel pores that are smaller in size and formed internal structural ordering at least in regions. While ensuring the ordering of molecules and pores of the original polymeric microparticles, the present invention allows for a second, larger pores within the polymeric microparticles, which can improve the permeability of the separation matrice and enable use in separation for larger molecular weight biomolecules, and thereby expanded the separation range of the polymeric microparticles in chromatographic analysis. **In** addition, ordered pore arrangement, which is formed locally inside the microparticle, provides excellent mechanical properties and separation effect.

## Description

### TECHNICAL FIELD

The invention relates to natural polymeric microparticles with porous structures, and more particularly to polymeric microparticles with dual size pores, and to a method of manufacturing such polymeric microparticles.

### BACKGROUND

In manufacturing biomedicine (such as vaccines, antibodies, recombinant proteins, gene therapy vectors, etc.), several chromatographic separation steps are often required to remove various contaminants and impurities from the product, and chromatography media is a critical part of determining the separation efficiency.

Larger biological entities, such as proteins or viruses and other biomacromolecules, maybe rigid nanoparticles and have large molecular weight, and their diffusion in chromatography media is affected by hydrodynamic effects, steric hindrance and electrostatic interactions, and the interaction between solute and the chromatography media, and the like. Therefore, the diffusive and advective transport rate of the biomacromolecules in the polysaccharide microsphere may be influenced and can be 2-3 times slower than that outside of the beads in the free solution, which significantly impacts the separation efficiency. Therefore, to obtain a large adsorption capacity for biomacromolecules, it is common to use macroporous separation matrices. Above all, natural polysaccharide materials, such as agarose, cellulose and, glucan and the like, are expected to exhibit characteristics such as porosity and biocompatibility, and are often used as matrices for separating biomacromolecules, but larger pore generally results in lower rigidity and the chromatographic medium may collapse at a higher flow rate.

One approach to the problem is preparing polysaccharide gel beads with ordered pore structures. The result of using these new polysaccharide gel beads has been a dramatic increase in the separation efficiency of the protein. However, during the formation of the polysaccharide gel beads, the porous polysaccharide gel beads prepared by common crosslinking methods show smaller pores and and therefore limited separation range when used as solid phase in chromatography columns, which consequently does not meet the need for simultaneously separating larger and smaller macromolecules. However, according to known techniques, if pore-formers such as a carbonate, a metal oxide and hydrophilic non-reactive materials are introduced into the microspheres that are unable to form an ordered structure and then are removed by using an acid reagent, such problems as the release of heat and gas may appear. The internal structure of the microspheres is damaged or even collapsed if heat and gas in the microspheres cannot be removed promptly.

Accordingly, there is a need to provide polymeric microparticles with controlled, uniform size and simultaneously have macropores and an ordered internal gel pores distribution and the overall structure of the microsphere is not affected by acid treatment, to improve separation efficiency of the chromatography column during the chromatographic separation and thus reduce the protein separation time.

### SUMMARY

This invention aims to provide polymeric microparticles with two sets of pores, each having distinctively different sizes distributed inside the beads, wherein at least sections of the gel pores have internal structural ordering.

The embodiment and purpose of the present application are hereinafter described and illustrated, given by examples combined with systems, tools, and methods. These examples are just exemplary and explanatory rather than limitation. In various embodiments, one or more market requirements have been met by the present invention, while other embodiments are directed to other improvements.

The primary objective of the present application is to provide a polymeric microparticle with dual-size pores which are formed by crosslinking at least partially cross-linkable oligomer materials, and the rigid nanoparticles at least partially form a substantially ordered structure, such as orientational order in the polymeric microparticle.

Another object of the present application is to provide a polymeric microparticle with dual-size pores. The polymeric microparticles have two sets of pores with distinctive sizes distributed inside, wherein the first set of pores are macropores larger in size. The second set of pores are gel pores that are smaller in size and form internal structural ordering at least in regions.

Another object of the present application is to provide a polymeric microparticle with dual-size pores, further comprising polysaccharide compounds for pressure resistance and structural support, besides the above rigid nanoparticles for providing an ordered structure.

Another object of the present application is to provide a method for preparing the polymeric microparticle with dual-size pores. The method above can obtain the essential structure of the polymeric microparticles provided in the present application.

For the purposes of the application, the application provides a polymeric microparticle with dual-size pores, which is formed by crosslinking at least partially cross-linkable oligomer materials, including rigid nanoparticles, wherein at least one of the rigid nanoparticles has a non-spherical shape in a solution. The polymeric microparticles have two sets of pores having distinctive sizes distributed inside, wherein the first set of pores are macropores larger in size, and the second set of pores are gel pores that are smaller in size and form internal structural ordering at least in regions, wherein the pore diameter of the macropores ranges from 2-20µm, the pore diameter of the gel pores range from 1-1000 nm.

As a further improvement of the application, the rigid nanoparticle is a biomacromolecule.

As a further improvement of the application, the shape of the non-spherical rigid nanoparticles is rod-shaped, strip-shaped, sheet-shaped, needle-shaped, or linear with its feature direction along the direction of the longitudinal axis of the molecule.

As a further improvement of the application, the shape of the non-spherical rigid nanoparticles is disk-shaped, with its feature direction normal to the disk.

As a further improvement of the application, at least in a substantially ordered area, at least a portion of the gel pore and at least a portion of its adjacent one has a certain correlation with the arrangement of their directions and positions.

As a further improvement of the application, at least in a substantially ordered area, at least a portion of the gel pore is substantially parallel, fan-shaped, or spirally arranged with at least a portion of its adjacent one.

As a further improvement of the application, the polymeric microparticles further comprise a polysaccharide compound having no obvious non-spherical shape in a solution, and the polysaccharide compound and the rigid nanoparticles are copolymerized to form the polymeric microparticles.

The application also discloses the use of the above-mentioned polymeric microparticles with dual-size pores as stationary phases for chromatographic separations.

On the other hand, the present application also discloses a method for preparing the aforementioned polymeric microparticles, comprising mixing and emulsifying an aqueous phase containing the rigid nanoparticles and the pore-former and an oil phase which are mutually insoluble with the aqueous phase, after curing to crosslink, the pore-former is then removed to give the porous polymeric microparticles containing both the ultra large pores and ordered pores.

As a further improvement of the application, the above preparation method specifically comprises the following steps of:
a) dispersing the rigid nanoparticles and the pore-formers into water to form a dispersed phase solution;
b) dispersing the dispersed phase solution in a continuous phase containing an emulsifier to form emulsion droplets containing the rigid nanoparticles;
c) adding a cross-linking agent to the thus-obtained product to cross-link the rigid nanoparticles in the emulsion droplets to form the polymeric microparticles after the continuous phase solvent is removed;
d) removing the pore-former by washing to obtain the polymeric microparticles with dual-size pores.

As a further improvement of the application, the rigid nanoparticle is a biomacromolecule.

As a further improvement of the application, wherein step a) hereinbefore described further comprises the step of adding a polysaccharide compound.

As a further improvement of the application, the pore-former is selected from inorganic salts, single-stranded RNA viruses or metallic oxides.

As a further improvement of the application, at least one member of the pore-former is selected from the group consisting of magnesium carbonate, barium carbonate, calcium carbonate, aluminium oxide, and tobacco mosaic virus.

As a further improvement in the application, the emulsifier is selected from a nonionic surfactant or anionic surfactant.

### Technical effects or advantages:

The polymeric microparticles with dual-size pores according to the present application ensure at least the local molecular and pore ordering in the original polymeric microparticles, which can improve the permeability of the separation matrice and enable use in separation for larger molecular weight biomolecules, and thereby expanded the separation range of the polymeric microparticles in chromatographic applications. Meanwhile, according to the polymeric microparticles disclosed in the present application, the rigid nanoparticles at least partially form a substantially ordered structure in its interior, so as to form a corresponding pore structure which is at least partially ordered, and the separation effect can be effectively improved when used as a stationary phase for chromatographic separation. When used as a stationary phase in chromatographic separation, the uniform radial arrangement structure formed inside the polymeric microparticles may enable the microparticles having excellent mechanical properties and separation effect.

### BRIEF DESCRIPTION OF THE DRAWINGS

FIG. 1 is a schematic view of a polysaccharide microsphere according to prior art and shows in magnified scale a detail of a part A of FIG. 1;
FIG. 2 comprises three pictures, in which FIG. 2(a) is a schematic view of a rod-shaped structure of biomacromolecules and a structural formula of an example, and FIG. 2(b) is a schematic view of an arch-shaped (banana-shaped) structure of a biomacromolecule and a structural formula of an example, and FIG. 2(c) is a schematic view of a disc-shaped structure of biomacromolecules and a structural formula of an example;
FIG. 3 comprises three pictures, in which FIG. 3(a), (c), (e) ,and (g) are schematic views of the arrangement of CNC nanorods when the concentration of the CNC dispersion is less than 3%, 3.5% -4%, 4%, and 5%, respectively, and FIG. 3(b), (d), (f) ,and (h) are crossed polarized microscopy images when the concentration of the CNC dispersion is less than 3%, 3.5% -4%, 4%, and 5%, respectively.
FIG. 4 is a schematic view of a process for forming emulsion droplets of lyotropic liquid crystals.
FIG. 5 shows two binding modes of rigid nanoparticles in polymeric microparticles;
FIG. 6 is a schematic view of the arrangement of chirality formed by biomacromolecules in polymeric microparticles;
FIG. 7 comprises four pictures, in which FIG. 7(a) is a cross-sectional view of a polymeric microparticle disclosed by the present application, and FIG. 7(b) shows in magnified scale a detail of a part B of FIG.7(a), and FIG. 7(c) shows in magnified scale a detail of a part C of FIG.7(a), and FIG. 7(d) is the SEM photographs of the polymeric microparticles in some embodiments;
FIG. 8 comprises four pictures, in which FIG.8(a) is the radial configuration of the polymeric microparticles, and FIG. 8(b) is the bipolar configuration of the polymeric microparticles and FIG. 8(c) is the loop conformation of the polymeric microparticles, and FIG.8(d) is the crossed polarized microscopy image of the polymeric microparticles with the radial configuration;
FIG. 9 shows a block diagram of a method for preparing polymeric microparticles according to embodiments;
FIG. 10 comprises six pictures, in which FIG.10(a), (c) and (e) are schematic views of the internal structures of polymeric microparticles which have no order, partial order and complete order, respectively, and FIG.10(b), (d) and (f) are the crossed polarized microscopy images of the polymeric microparticles which have essentially no order, partial order and complete order, respectively.
FIG. 11 is a schematic view of the polymeric microparticle's (a) crossed polarized microscopy images before the acid treatment and (b) crossed polarized microscopy image after the acid treatment, which is prepared according to Embodiment 1 of the present application.
FIG. 12 is a schematic view of the polymeric microparticle's (a) scanning electron microscopy image before the acid treatment and (b) scanning electron microscopy image after the acid treatment, which is prepared according to Embodiment 1 of the present application.
FIG. 13 is a schematic view of the polymeric microparticle's (a) scanning electron microscopy image before the acid treatment and (b) scanning electron microscopy image after the acid treatment, which is prepared according to Embodiment 2 of the present application.
FIG. 14 is a schematic view of the polymeric microparticle's (a) crossed polarized microscopy image before the acid treatment and (b) crossed polarized microscopy image after the acid treatment, which is prepared according to Embodiment 3 of the present application.
FIG. 15 is a schematic view of the polymeric microparticle's (a) scanning electron microscopy image before the acid treatment and (b) scanning electron microscopy image after the acid treatment, which is prepared according to Embodiment 3 of the present application.
FIG. 16 is a schematic view of the polymeric microparticle's scanning electron microscopy image after washing until neutral, which is prepared according to Embodiment 4 of the present application.
FIG. 17 is a schematic view of the polymeric microparticle's scanning electron microscopy image after the acid treatment, which is prepared according to Comparison Example 1 of the present application.

### DETAILED DESCRIPTION OF EMBODIMENTS

In order to make the objects, technical solutions, and advantages of the present application clearer, the technical solutions of the present application will be clearly and completely described in the following section with reference to the embodiments of the present application. It is apparent that the described embodiments are a part of the embodiments of the present application rather than all of the embodiments. All other embodiments obtained by those skilled in the art based on the technical solutions and embodiments provided by the present application and without the creative work are all within the scope of the present application.

Hereinafter, the present invention will be described in detail.

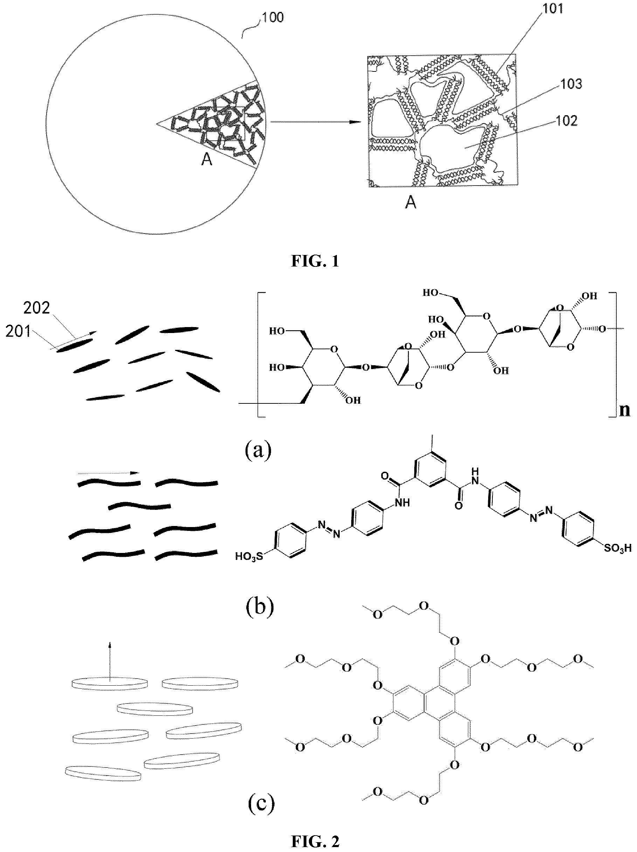

The preparation method for porous microspheres known in the prior art which is suitable for use in a chromatography column consists of dispersing polysaccharide molecules (such as agarose) in water. Moreover, the polysaccharide-containing tiny aqueous droplets suspended in the oil phase are formed by appropriate emulsification techniques. Referring to FIG. 1, after the temperature of the emulsion is cooled, the single chain of the polysaccharide compound 101 forms a double helices structure, and then pore 102 are formed between the molecular beams, and finally, the microspheres 100 are formed with the aid of crosslinking agent 103. As the polysaccharide molecules are arranged completely in a disordered manner in water, the internal pores of the microspheres thus formed are also proved to be arranged ordered randomly, and given the inherent soft characteristics of the polysaccharide molecules, the microspheres cross-linked are usually relatively soft.

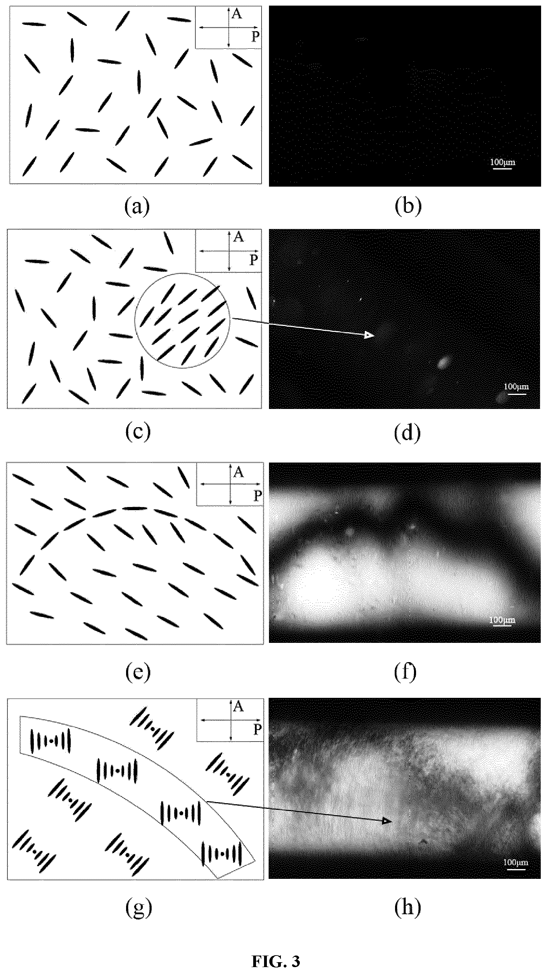

Many biomacromolecules in nature are independent or dispersed in water to present a non-spherical, symmetrical, rigid form, as shown in FIG.2, including a rod-like shape shown on the left side of FIG. 2(a), such as tobacco mosaic virus (TMV) , deoxyribonucleic acid (DNA), cellulose nanocrystals (CNC, schematic and structural formulas shown in the right side of FIG.2(a)), and a bow-like shape(banana-shaped) shown on the left side of FIG. 2(b), such as P52C molecule(schematic and structural formulas shown in the right side of FIG.2(b)), and 2, 3, 6, 7, 10, 11 - six (1,4,7 - trioxylalkyl-benzo [ 9,10 ] phenanthrene 7) (TP 6EO2M, schematic diagram and structural formula shown in the right side of FIG. 2 (C)) having a disc shape shown on the left side of FIG. 2(c), and the like. The tobacco mosaic virus is a rigid rod-like biological nanoparticle that is widely studied in liquid crystal physics. According to the lyotropic liquid crystal theory, when such rigid nanoparticles are dispersed in a solvent, the rigid nanoparticles may be arranged disorderly in the solvent or in an ordered molecular arrangement of some kind of lyotropic liquid crystals such as a nematic phase (such as a tobacco mosaic virus nanomaterial), a near-crystalline phase, a cholesteric phase, and a columnar phase liquid crystal, depending on the concentration and the characteristics of the rigid nanoparticles. Liquid crystal materials ordered usually exhibit optical birefringence, so the ordered droplets or solvents containing non-spherical biomacromolecules are clearly visible under a polarizing microscope, and a common liquid crystal conformation is presented, as shown in FIG. 3.

More particularly, in accordance with the spirit of this application, using non-spherical rigid nanoparticles and pore-formers, it would be possible to prepare the porous polymeric microparticles having both large flow-through macropores and gel pores where the orientation of the nanoparticles are ordered and the pore structures are controllable, and the microparticles have excellent mechanical properties.

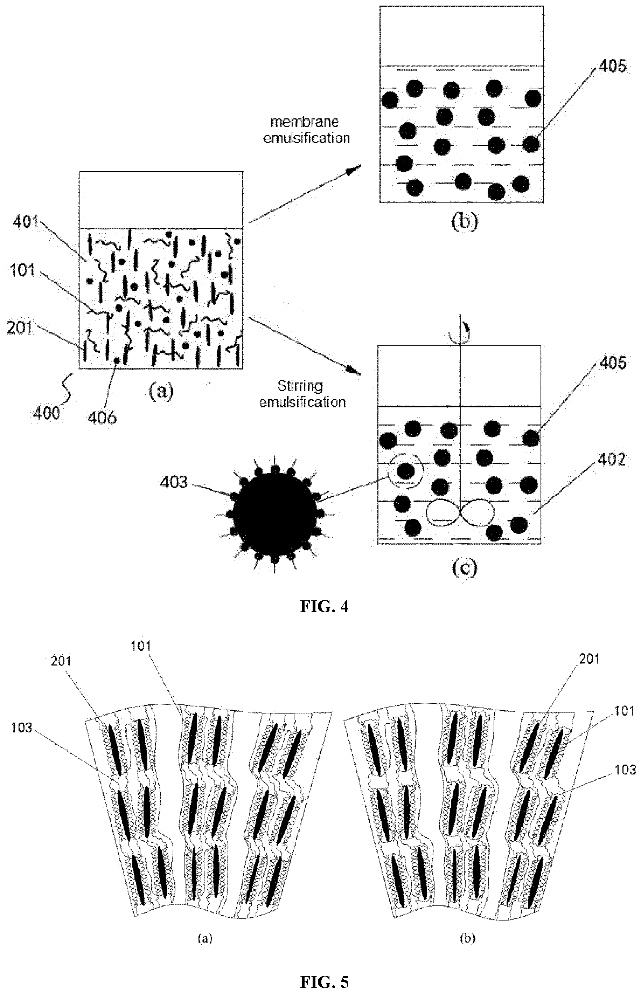

As shown in FIG. 4, in accordance with the spirit of this application, an appropriate amount of rigid nanoparticles 201 having non-spherical shape are uniformly dispersed in the solvent 401 alone or with an appropriate amount of amorphous monomers or oligomers 101 and pore-formers 406, to form the lyotropic liquid crystal solution 400 having local or overall molecular orientation ordering as shown in FIG. 4 (a). The lyotropic liquid crystal solution 400 forms an emulsion containing the lyotropic liquid crystal droplets 405 suspended in the solvent 402 by an appropriate emulsification method, including the membrane emulsification shown in FIG. 4 (b) or stirring to emulsify (FIG. 4 (c)) by adding a solvent 402 immiscible with the lyotropic liquid crystal solution and the emulsifier 403, etc. The monomers or oligomers and the rigid nanoparticles with polymerizable functional groups are polymerized to form a polymer network structure, thereby forming porous polymer microspheres which contain rigid nanoparticles and the rigid nanoparticles with at least partially formed ordered structures in them. The rigid nanoparticles may be physically wrapped in the polymer microspheres by the surrounding polymer in the polymeric microparticles 410, as shown in FIG. 5 (b). They may also directly participate in the cross-linking reaction and become part of the polymer via chemical bonding, as shown in FIG. 5 (a).

By the spirit of this application, when the rigid nanoparticles are biomacromolecules, it would be possible to prepare the porous polymeric microparticles to have at least partially ordered molecules with both micron-size macropores and nano-sized small pores which are arranged in order.

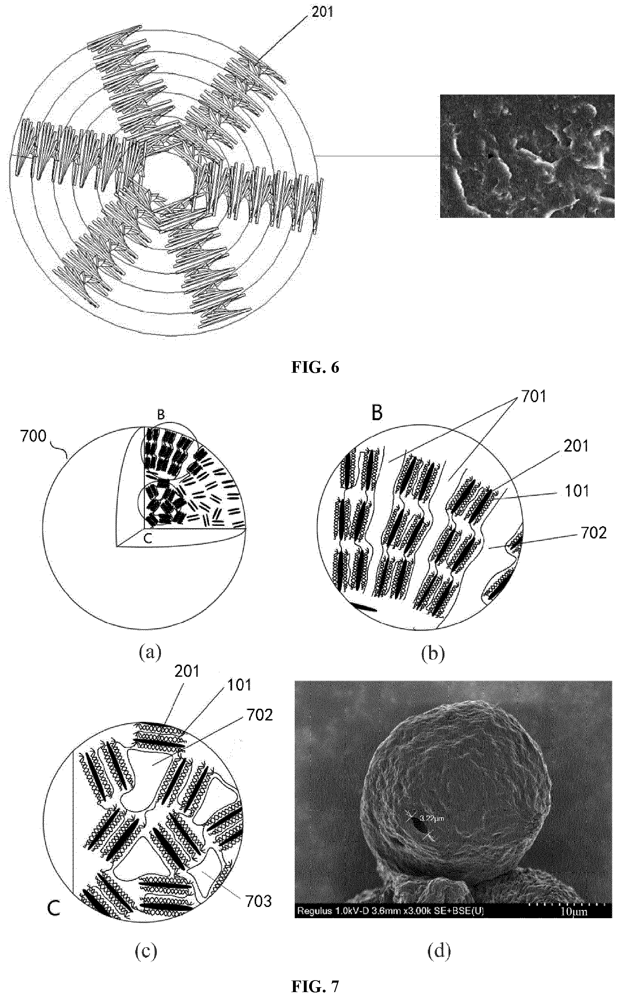

Specifically, cellulose nanocrystals CNC having a suitable aspect ratio and size distribution are biomacromolecules that have certain rigidity in their solvent water and can form a lyotropic liquid crystal phase. In accordance with the spirit of this application, when the biomacromolecules are cellulose nanocrystals (CNC) and as its concentration reaches a critical concentration, the CNC molecules may be self-assembled to form an ordered structure, thereby showing the liquid crystal phase. As shown in FIG. 3 (a, b), for the CNC nanomaterial with a specific aspect ratio, there is no orientation ordering of the CNC nanomaterials for CNC concentration below 3%, then it does not have a liquid crystal phase and the corresponding solvent does not show the birefringence characteristic under a polarization microscope, and the picture presents a uniform dark state. As shown in FIG. 3 (c, d), when the concentration of the CNC dispersion is between 3.5% and 4%, a fluctuation of the ordered domain may occur due to insufficient or fluctuation in local concentrations of the dispersion. As shown in FIG. 3 (e, f), biomacromolecules can be regularly arranged in a liquid crystal phase state as the concentration of CNC dispersion is higher than the critical concentration of 4% to form an ordered structure. As shown in FIG. 3 (g, h), since the CNC biomacromolecules have chiral characteristics, the chiral characteristics of the biomacromolecules can be clearly shown to form a cholesteric phase molecule arrangement with a spiral structure when the concentration of the dispersion is about 5%. When the concentration of the CNC dispersion is higher than 6%, the arrangement of the biomacromolecules is more orderly. When the aspect ratio and uniformity of the CNC nanomaterial are changed, the corresponding critical concentration is also changed. At the same time, since the cellulose nanocrystals (CNC) biomacromolecules are chiral, the ordered molecular structures can form a liquid crystal molecule arrangement with a helical structure, as shown in FIG. 6.

FIG. 7 is a polymeric microparticle with dual-size pores according to the present application. Specifically, FIG. 7 (a) shows a diametrical cross-section of the partial interior space of the polymer microsphere. The rigid nanoparticles may remain an ordered structure at least in part after crosslinking, and due to the influence of the molecular arrangement, the pore structure formed may also be partially ordered, as shown in FIG. 7 (b). The rigid nanoparticle solution shown in FIG. 7 (a) may be emulsified to form droplets when it may also be in a local ordering state, and the rigid nanoparticles ( i.e., biomacromolecules) within the emulsion also similarly tend to be partially ordered as shown in FIG. 7 (a). When the temperature of the emulsion is lowered, the rigid nanoparticles 201 retain the alignment and are further cross-linked to form polymeric microparticles. The internal structure at least partially retains the previously ordered conformation, and the pore-formers will tend to take positions around the rigid nanoparticles and the polysaccharide molecules, and at the same time, as shown in FIG. 7 (b), the ordered gel pores 701, and the macropores 702 formed between the rigid nanoparticles inherit the same ordered conformation. After further pore-former removal, micron-size flow-through macropores can be formed around the ordered gel pores, thereby forming the polymeric microparticles 700 simultaneously having micron-size macropores and nano-sized small pores, which are arranged in order. The phrase "substantially ordered" refers to that at least in a substantially ordered area, at least a portion of the pore and at least a portion of its adjacent one has a certain correlation with the arrangement of their directions and positions. Specifically, at least in a substantially ordered area, at least a portion of the pore is substantially parallel, fan-shaped, or spirally arranged with at least a portion of its adjacent one. As a preferred embodiment, the pore diameter of the macropores larger in size is 2-20 µm, and the pore diameter of the gel pores that are smaller in size is 1-1000 nm. As shown in FIG. 7(d), the pore diameter of a macropore is 3.22 µm.

**In** accordance with the spirit of this application, the substantially ordered structures of rigid nanoparticles in the emulsion may include one or more regions by controlling its concentration. Meanwhile, the molecular arrangement between the plurality of regions may not be correlated, correlated, or partially correlated. Moreover, the substantially ordered structures may be overall ordered, or may be partially ordered. When in overall ordered form, in a substantially ordered region, the distribution of the feature direction of the rigid nanoparticles is any one of substantially along the particle radius direction, along the particle bipolar axis direction, or in a plurality of concentric circles inside the microparticles. When in partially ordered form, in a substantially ordered region, the feature direction of the rigid nanoparticles is substantially parallel, fan-shaped, or spirally arranged.

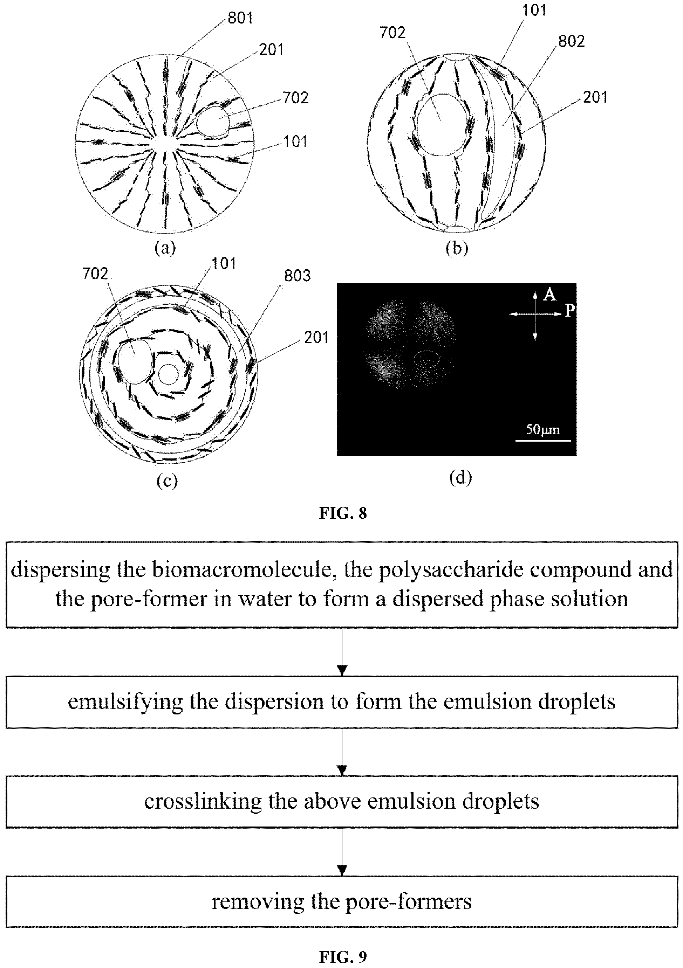

Within the overall ordered range, some special conformations may be formed structurally due to these substantially ordered structures, including the radial configuration (the feature direction is arranged in order along the radius direction), and first ordered pores 801 are formed with its directions point at the circle center, as shown in FIG. 8 (a), and the bipolar configuration as shown in FIG. 8 (b) (the feature direction is arranged in order along the bipolar direction), and second ordered pores 802 are formed inside which is along the direction of the bipolar axis, and the loop conformation as shown in FIG. 8 (c) (the feature direction is in concentric circle arrangement), and third ordered pores 803 are formed inside whose arrangement is a concentric circle arrangement. However, this disclosure is not limited thereto, and it may also be other ordered conformation. In addition, several macropores 702 were formed dispersively between the ordered pores. However, from the overall point of view, the presence of macropores does not affect the overall ordering of the gel pores. At the same time, due to the optical birefringence characteristic generally possessed by biomacromolecules, these special conformations show a special optical phenomenon under a polarizing microscope. For example, as shown in FIG. 8 (a), the feature direction of the biomacromolecules tends to be arranged in order along the radius direction in the emulsion drops, and the interior structure and the pores of the polymeric microparticles formed thereby also tend to be arranged in order along the radius direction, thus having a radial configuration and showing an optical anisotropy of Maltese black cross under a polarizing microscope. At the same time, when the pore-former is mixed into the emulsion droplets, the structure arrangement of the biomacromolecules in the emulsion droplets will be partially affected, which results in changes in the local orientation, as shown in FIG. 8(d).

Within the partially ordered range, as shown in FIG. 7 (a), the external region of the polymeric microparticles comprises multiple parts B, as shown in FIG. 7(b) and multiple parts C as shown in FIG. 7(c). Part B is a local region with the feature direction of the rigid nanoparticles substantially ordered, and part C is a disordering region for the feature direction of the rigid nanoparticles. Nano-sized ordered gel pores 701 and nano-sized disordered pores 703 are formed inside the polymeric microparticles at the same time. Meanwhile, the macropores 702 were formed dispersively both in part B and part C. Even though the polymeric microparticles do not have a specific conformation, the feature directions of the polymeric microparticles are still regularly arranged in a small area, and color development can still be observed under a polarizating microscope.

In accordance with the present invention, it would be possible to prepare the biomacromolecule droplets of different sizes and partially ordered structures, which are then crosslinked to form the polymeric microparticles that are at least partially ordered on the molecular and pore structural scale. **In** a preferred embodiment, the average particle size of the polymeric microparticles in the solvent, which is usually an aqueous solvent, is 1-500 µm. More preferably, the average particle size is 5-150 µm. When used as fillers for chromatography columns, very small particles will lead to a high back pressure, and very large particles will lead to low separation efficiency.

**In** accordance with the spirit of this application, the polymeric microparticle 700 which is formed by crosslinking at least partially cross-linkable oligomer materials including rigid nanoparticles 201, wherein at least one of the rigid nanoparticles has a non-spherical shape in a solution. As shown in FIG. 2(a), the rigid nanoparticles 201(i.e., biomacromolecules) is a rod shaped molecule with a feature direction 202 in the direction of the longitudinal axis of the molecule. As shown in FIG. 2(b), the biomacromolecule is a bow-like shaped (banana shaped) molecule with its feature direction in the direction of the longitudinal axis of the molecule, or as shown in FIG. 2(c), the biomacromolecule is a disk-like shaped molecule with a feature direction perpendicular to the disk, the biomacromolecule can also be but is not limited to the form of slice, needle or wire, other suitable non-spherical biomacromolecule can also be used.

Biomacromolecules with or without spherical shapes are selected from at least one of a polypeptide (e.g., insulin, growth hormone), a protein (e.g., chloroplastin, collagen, etc.), a nucleic acid (e.g., DNA), a polysaccharide (e.g., cellulose, chitosan), and a lipid (e.g., monoglyceride, phospholipid, glycolipid, steroid, etc.). These biomacromolecules are commonly found in organisms, and are mostly in a rod shape or a flat shape in the solution. In a preferred embodiment, as shown in FIG. 6, the biomacromolecules may or may not be chiral. Further, the biomacromolecules with chiral characteristics include left- and right-handed biomacromolecules, and the formed liquid crystal phases may form a cholesteric helical structure. As illustrated in the corresponding part of the SEM photographs with arrow in Fig.4, the broken parts of the polymeric microparticles exhibit a helical ribbon-like internal structure. One specific embodiment of the present application is a biomacromolecule cellulose nanocrystal (CNC), and the structural formula is shown below.

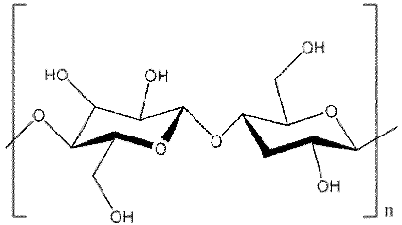

The rod-shaped biomacromolecules have a larger aspect ratio, and are easier to form a liquid crystal state. As a further preferred embodiment, the cellulose nanocrystal has a length of 20-1000 nm, a width of 2-100 nm, and an aspect ratio of 1:5-1: 200.

As shown in FIG. 7 (b), the polymeric microparticles may further include a polysaccharide compound 101 having a non-spherical shape, and these polysaccharide compounds and the biomacromolecules are copolymerized to form the polymeric microparticles. These polysaccharide compounds are selected from at least one member of agar, agarose, glucan, starch, chitosan and trehalose. In detailed embodiments of the present application, the polysaccharide compound is agarose with the following structural formula:

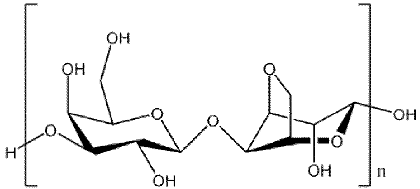

The polysaccharide compound is a gel-like dispersion that can flow prior to emulsification. After the dispersion is emulsified into emulsion droplets, as shown in FIG. 7 (b), a double helix of the polysaccharide compound 101 was formed by its single chain through the processes of cooling, curing, aging, and the like, and therefore forms a bundled molecular structure that ultimately forms soft but stable solid porous particles. Although these polysaccharide compounds are not self-orderly arranged in the solution, they will arrange along with the biomacromolecules because of a variety of interactions between these polysaccharide compounds and the biomacromolecules, including hydrogen bonds, and eventually form an ordered structure. At the same time, in some cases, the biomacromolecules are partially ordered to form a liquid crystal state at a specific concentration, and the polysaccharide compounds will be arranged according to the arrangement of biomacromolecules as a template. These polysaccharide compounds and biomacromolecules are further copolymerized under the assistance of a cross-linking agent to form a stable particle structure but not to destroy the ordered structure of the polymeric microparticles, thereby further improving the pressure resistance of the formed polymeric microparticles. Meanwhile, in the preparation process of the polymeric microparticles, the polysaccharide compound (such as agarose) can solidify the emulsified emulsion by cooling to provide structural support for the subsequent cross-linking polymerization, thereby simplifying the preparation process.

In accordance with the spirit of this application, the present application further provides a method for preparing the polymeric microparticles, and the specific process is described as follows:

As shown in FIG.9, an embodiment of the present application may include a method of preparing the polymeric microparticles. The method may include dispersing a rigid nanoparticle, a polysaccharide compound and a pore-former to form a dispersion, specifically, dispersing the biomacromolecule, the polysaccharide compound and the pore-former in water to form a dispersed phase solution. By controlling the parameters of the specific biomacromolecule, including its aspect ratio, size distribution, etc. and the concentration in water thereof, it is possible to control the biomacromolecules to form an ordered or disordered state in the solvent. Further, mechanical property including pressure resistance of the polymeric microparticle can be further regulated by adjusting the mass ratio of the biomacromolecule and the polysaccharide compound. The porosity of the macropores can be further adjusted by adjusting the amount of the pore-forming agent.

In some embodiments, the pore-former is selected from inorganic salts, single-stranded RNA viruses or metallic oxides. Further, at least one member of the pore-former is selected from the group consisting of magnesium carbonate, barium carbonate, calcium carbonate, aluminium oxide, and tobacco mosaic virus. In particular embodiments, the pore-former is calcium carbonate, and the calcium carbonate microparticles are non-toxic which makes it possible to generate large flow-through macropores while maintaining the ordered internal gel pores of the polymeric microparticle; Further, the the mass percentage concentration of the pore-former in the dispersed phase solution is 0.01% to 1%.

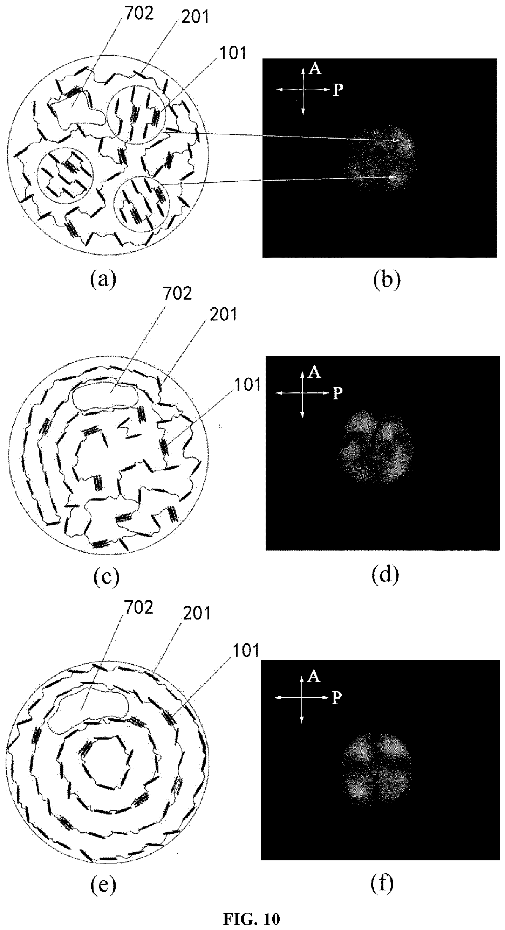

Second, the method further comprises the step of emulsifying the dispersion to form the emulsion droplets. Various methods for emulsification exist, including membrane emulsification. The membrane emulsification method refers to a method of forcing a dispersion phase into a continuous phase directly through the pores of a microporous membrane. Then the emulsion droplets are formed and extruded at the end of the pores in a process of emulsification. Another common emulsification method involves dispersing a dispersed phase solution formed by a co-dispersing solution of a biomacromolecule and a polysaccharide compound in a continuous phase containing emulsifying agents to form the emulsion droplets containing biomacromolecules. The emulsifiers can facilitate the formation of dispersions of emulsion droplets. Meanwhile, it can also assist with arranging the biomacromolecule in order and preparing the emulsion droplets with different orientational effects by controlling the temperature and orientation time. It can also further prepare the polymeric microparticles with the corresponding orientation effect. As shown in FIG. 10 (a) and FIG. 10 (b), most of the nanorods in the particles are arranged in a disordered configuration, and the nanorods in a few regions are ordered. Only a small part of the sheet-like region displays a bright state under a polarization microscope, and then the whole picture presents an uneven, dark state. As shown in FIG. 10 (c) and FIG. 10 (d), part of the nanorods in the particles are arranged in a disordered configuration, while other regions are ordered, and then the unobvious birefringence property begins to be presented. As shown in FIG. 10 (e) and FIG. 10 (f), the nanorods in the particles are arranged in a plurality of concentric circles or may be arranged in other overall ordered manner, and the polymer microspheres show a radial optical anisotropy (Maltese Black Cross) under polarized light. Meanwhile, as shown in FIG. 10 (e) and FIG. 10 (f), a plurality of macropores 702 were formed dispersively between the disordered gel pores and between the ordered gel pores. As a preferred embodiment, the mass concentration of the emulsifier in the continuous phase is 1% -25%. The ratio of the dispersed phase to the continuous phase is preferably 1:1 to 1:15. The dispersion modes may be a common emulsification-diffusion process, such as the stirring, ultrasonic, and vibration methods.

In some embodiments, the emulsifying agents may be sorbitol esters-based (SPAN) surfactants such as sorbitan monopalmitate (SPAN 40), sorbitan monostearate (SPAN 60), sorbitan tristearate (SPAN 65), sorbitan monooleate (SPAN 80), sorbitan trioleate(SPAN 85) and the like. It can also be Tween surfactants such as Tween20, Tween 40, Tween 60, Tween 80 or Tween 85. It can further be a cetyl alcohol, polyglyceryl polyricinoleate (PGPR), and the like. The continuous phase is an oily substance that is incompatible with the water and can dissolve the emulsifier, such as linear alkanes (such as n-hexane, n-hexadecane, etc.), liquid paraffin, animal or vegetable greases (such as soybean oil). Further, the mass concentration of the emulsifier in the continuous phase is 1% -25%. The ratio of the dispersed phase to the continuous phase is preferably 1:1 to 1:15.

Again, the method comprises the step of crosslinking the above emulsion droplets. The specific process is to add a cross-linking agent to the emulsion droplets formed in the previous step to crosslink biomacromolecules in the emulsion, finally form the polymeric microparticles, wherein the crosslinking agent is selected from epoxide, diacid chloride or halogen compound. In one specific embodiment of the present application, the epoxide is glycerol ether, a small-molecule organic compound.

When the crosslinking agent is an epoxide, the crosslinking process is as follows:

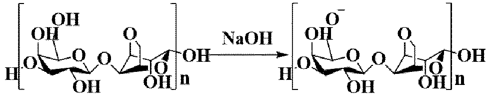

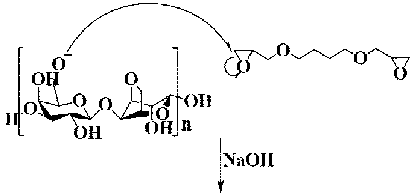

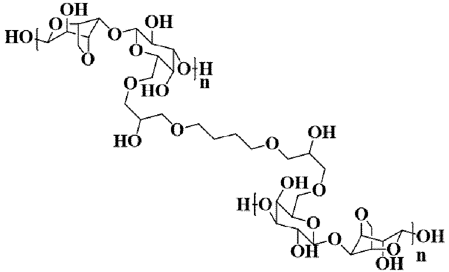

When the crosslinking agent is a halogen compound, the crosslinking process is as follows:

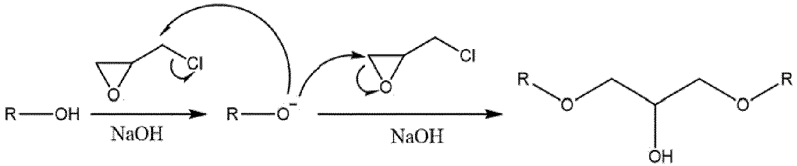

Since the surface of the biomacromolecules have numerous hydroxyl groups, which can be further capable of undergoing a cross-polymerization reaction with the aid of the crosslinking agent, and finally form stable polymeric microparticles. At the same time, the pore structure formed in the emulsification process is reinforced. Since the ordered molecular arrangement of the biomacromolecules before crosslinking, the finally formed pore also tends to have the same ordered molecular arrangement that further forms an ordered internal structure and pore distribution. As a preferred embodiment, cross-linking is carried out in basic conditions, and that makes the crosslinking agent more conducive.

Finally, the method further includes the step of removing the pore-formers, specifically, washing and removing the pore-formers to obtain the polymeric microparticles with dual-size pores. Based on ensuring the original ordered pore of the polymeric microparticles, a certain amount of micron-size flow-through macropores are additionally arranged in the polymeric microparticles. The mobile phase flows in the manner of a convection current within the macropores while it flows in a spreading manner within the gel pores.

The above polymeric microparticles with a porous structure may be used for biochemical separation, particularly as the stationary phase for column chromatography. When a mobile phase passes through the column, the constituents in the mobile phase will interact with the stationary phase. The size of the constituents, the pore size and its distribution of the stationary phase, and the affinity of the constituents in the mobile phase to the stationary phase all contribute to when and how a specific component passes through the column, and thereby achieving separation of the contents in the mobile phase Since the microparticles, according to the present invention, are comprised of biomacromolecules, chromatography columns made using such microparticles show good bio-compatibility with bio-materials such as proteins to be separated. Additionally, due to the internal ordering of the molecules and pores in the microparticles, the constituents in the mobile phase may have a less tortuous path, there can be an improved separation efficiency when compared with like columns using microbeads with no internal structural ordering. In a further aspect, the added macroporous structure can improve permeability of the separation matrice and expand the separation range of the polymeric microparticles, and thereby results in a two-pronged effect in improving the separation efficiency.

In order to further achieve the object of the present application, the present application further provides another application scenario of the above polymeric microparticles; specifically, an in-situ polymerization is conducted by adding a cross-linking agent directly rather than performing emulsification after the dispersed phase solution is formed, the output of which can be used as stationary phase for monolithic media chromatography.

The structure, optical performance, and preparation method of the polymeric microparticles will be described in detail below with reference to specific embodiments. Unless otherwise specified, mass ratio between different components will be used in the exemplary embodiments described below.

### Example 1

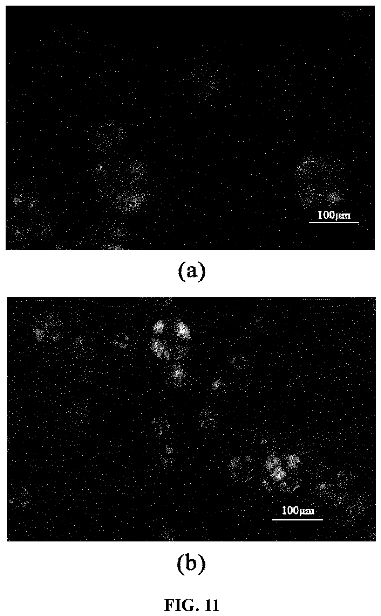

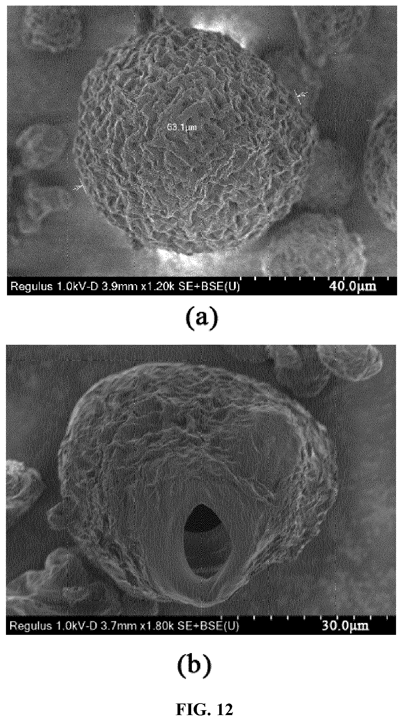

0.6 grams of cellulose nanocrystals, 0.045 grams of calcium carbonate powder and 0.3 grams of agarose were dispersed in 14.1 grams of water during the reaction, and then subjected to disintegration in an ultrasonic disruptor for 10 min. The mixture was stirred at 80°C, forming a suspension. The suspension was poured into 150 grams of liquid paraffin containing SPAN80 (10 percent by mass concentration) and were emulsified by stirring at 90°C for 5 mins, and cooled to form the dispersion containing cured emulsion droplets. The dispersion was washed to eliminate continuous phase solution and the measured gel product after the weighing was added with 10 mL of water, and then the crosslinker 1,4-butanediol diglycidyl ether having a proportion of 40 percent by mass to the mass of the gel product was added and stirred for 4 hours. The system was added with 500 microliters of aqueous solution containing 40 wt% sodium hydroxide and 5 wt% sodium borohydride, and the mixture was stirred for 12 hours. The system was added with epichlorohydrin having a proportion of 50 percent by mass to the mass of the reaction mixture, and 1500 microliters of aqueous solution containing 40 wt% sodium hydroxide were mixed and added to the reaction mixture, and the mixture was stirred for 12 hours. The resulting polymeric microparticles were washed until pH neutral, and then were soaked in solutions of 1 mol/L HCl until no bubbles were generated. After sieving, the microparticles that have the particle size between 40µm and 150µm are retained. The optical conformation of the particles before and after soaking under a polarizing microscope was observed and a freeze dryer was adopted for shaping, and then the morphology and aperture characteristics are observed by scanning electron microscopy (SEM). As shown in FIG.11, the polymeric microparticles after acid washing show a radial optical anisotropy (Maltese Black Cross) under a polarizing microscope, demonstrating it has radial internal configuration and radial pore distribution. The SEM images in FIG.12 show that the particle size of the microparticles is 63.1 µm and a certain amount of micron-size flow-through macropores are also formed, while bulk morphology of the microparticles is well maintained.

### Example 2

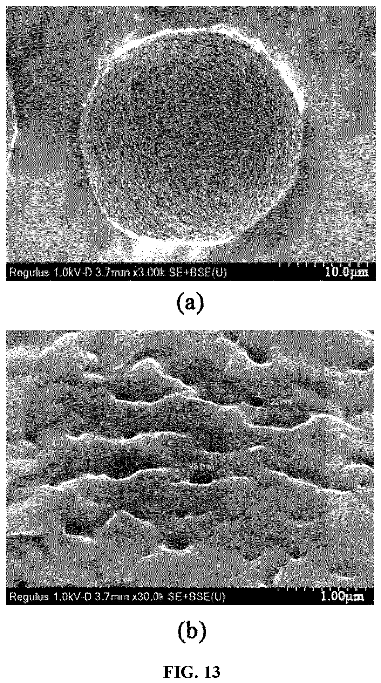

0.6 grams of cellulose nanocrystals, 0.075 grams of calcium carbonate powder and 0.3 grams of agarose were dispersed in 14.1 grams of water during the reaction and then subjected to disintegration in an ultrasonic disruptor for 10 min. The mixture was stirred at 80°C, forming a suspension. The suspension was poured into 150 grams of liquid paraffin containing SPAN80 (10 percent by mass concentration) and was emulsified by stirring at 90°C for 5 mins, and cooled to form the dispersion containing cured emulsion droplets The dispersion was washed to eliminate continuous phase solution and the measured gel product after the weighing was added with 10 mL of water, and then the crosslinker 1,4-butanediol diglycidyl ether having a proportion of 40 percent by mass to the mass of the gel product was added and stirred for 4 hours. The system was added with 500 microliters of aqueous solution containing 40 wt% sodium hydroxide and 5 wt% sodium borohydride, and the mixture was stirred for 12 hours. The system was added with epichlorohydrin having a proportion of 50 percent by mass to the mass of the reaction mixture, and 1500 microliters of aqueous solution containing 40 wt% sodium hydroxide were mixed and added to the reaction mixture, and the mixture was stirred for 12 hours. The resulting polymeric microparticles were washed until pH neutral, and then were soaked in solutions of 1 mol/L HCl until no bubbles were generated. After sieving, the microparticles that have the particle size between 40µm and 150µm are retained. The optical conformation of the particles before and after soaking under a polarizing microscope was observed. A freeze dryer was adopted for shaping, and then the morphology and aperture characteristics are observed by scanning electron microscopy (SEM). As shown in FIG.13, the pore sizes of the surface pores individually identified as 122nm and 281nm. It is challenging to observe the presence of macropores on the surface of the microparticles, which indicates that macropores cannot be generated in the case of a small amount of pore-formers.

### Example 3

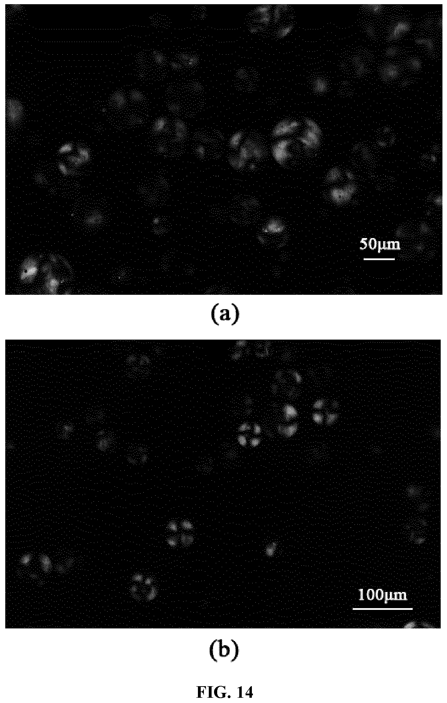

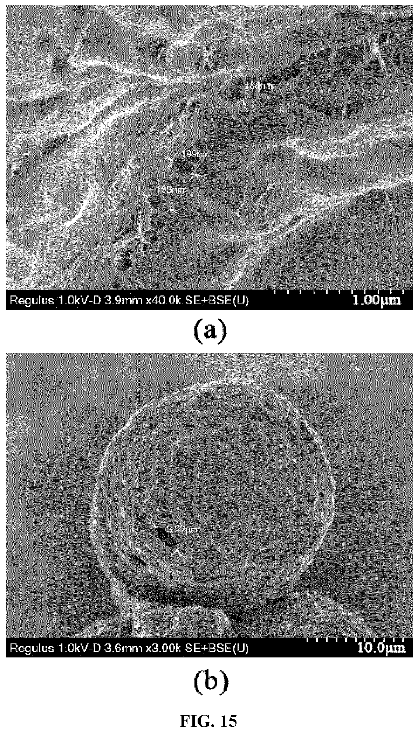

0.6 grams of cellulose nanocrystals, 0.03 grams of aluminium oxide powder and 0.3 grams of agarose were dispersed in 14.07 grams of water during the reaction and then subjected to disintegration in an ultrasonic disruptor for 10 min. The mixture was stirred at 80°C, forming a suspension. The suspension was poured into 150 grams of liquid paraffin containing SPAN80 (10 percent by mass concentration) and were emulsified by stirring at 90°C for 5 mins, and cooled to form the dispersion containing cured emulsion droplets. The dispersion was washed to eliminate continuous phase solution and the measured gel product after the weighing was added with 10 mL of water, and then the crosslinker 1,4-butanediol diglycidyl ether having a proportion of 40 percent by mass to the mass of the gel product was added and stirred for 4 hours. The system was added with 500 microliters of aqueous solution containing 40 wt% sodium hydroxide and 5 wt% sodium borohydride, and the mixture was stirred for 12 hours. The system was added with epichlorohydrin having a proportion of 50 percent by mass to the mass of the reaction mixture, and 1500 microliters of an aqueous solution containing 40 wt% sodium hydroxide were mixed and added to the reaction mixture, and the mixture was stirred for 12 hours. The resulting polymeric microparticles were washed until pH neutral and then were soaked in solutions of 1mol/L HCl until no bubbles were generated. After sieving, the microparticles that have a particle size between 40µm and 150µm are retained. The optical conformation of the particles before and after soaking under a polarizing microscope was observed. A freeze dryer was adopted for shaping, and then the morphology and aperture characteristics are observed by scanning electron microscopy (SEM). As shown in FIG.14, the polymeric microparticles after acid washing show a radial optical anisotropy (Maltese Black Cross) under a polarizing microscope, demonstrating it has radial internal configuration and radial pore distribution. As can be seen from the SEM images in FIG.15, the pore sizes of the microparticles before acid washing were individually identified as 188nm, 199nm and 195nm, and a certain amount of micron-size flow-through macropores are also formed after acid washing. In contrast, the bulk morphology of the microparticles is well maintained. The macropore diameter is 3.22 µm as shown in FIG.15.

### Example 4

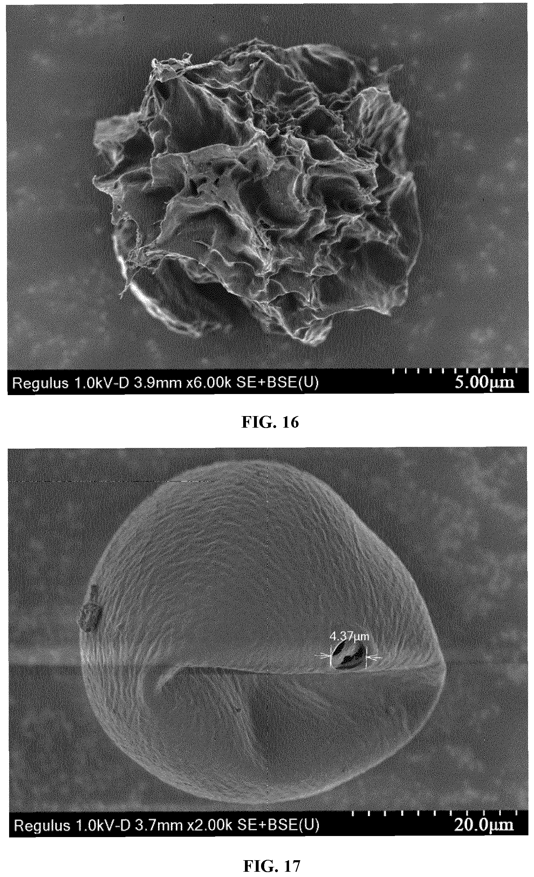

0.6 grams of cellulose nanocrystals, 0.2 grams of tobacco mosaic virus and 0.3 grams of agarose were dispersed in 14.07 grams of water during the reaction, and then subjected to disintegration in an ultrasonic disruptor for 10 min. The mixture was stirred at 80°C, forming a suspension. The suspension was poured into 150 grams of liquid paraffin containing SPAN80 (10 percent by mass concentration) and were emulsified by stirring at 90°C for 5 mins, and cooled to form the dispersion containing cured emulsion droplets. The dispersion was washed to eliminate continuous phase solution and the measured gel product after the weighing was added with 10 mL of water, and then the crosslinker 1,4-butanediol diglycidyl ether having a proportion of 40 percent by mass to the mass of the gel product was added and stirred for 4 hours. The system was added with 500 microliters of aqueous solution containing 40 wt% sodium hydroxide and 5 wt% sodium borohydride, and the mixture was stirred for 12 hours. The system was added with epichlorohydrin having a proportion of 50 percent by mass to the mass of the reaction mixture, and 1500 microliters of an aqueous solution containing 40 wt% sodium hydroxide were mixed and added to the reaction mixture, and the mixture was stirred for 12 hours. The resulting polymeric microparticles were washed until pH neutral. After sieving, the microparticles that have the particle size between 40µm and 150µm are retained. The optical conformation of the particles before and after soaking under a polarizing microscope was observed and a freeze dryer was adopted for shaping, and then the morphology and aperture characteristics are observed by scanning electron microscopy (SEM). As shown in Fig. 16, since the tobacco mosaic virus does not participate in the reaction, they were all washed off after sufficient washing, resulting in caverns. The tobacco mosaic virus has an average length of 300 nm and an average width of 20nm. Due to the large viscosity of the agarose and the CNC mixed system, the tobacco mosaic virus is difficult to disperse, and the microsphere structure altered after elution displayed larger pores.

### Comparative Example 1

0.9 grams of agarose and 0.03 grams of calcium carbonate powder were dispersed in 14.07 grams of water during the reaction and then subjected to disintegration in an ultrasonic disruptor for 10 min. The mixture was stirred at 100°C, forming a suspension. The suspension was poured into 150 grams of liquid paraffin containing SPAN80 (10 percent by mass concentration) and were emulsified by stirring at 90°C for 5 mins, and cooled to form the dispersion containing cured emulsion droplets. The dispersion was washed to eliminate continuous phase solution and the measured gel product after the weighing was added with 10 mL of water, and then the crosslinker 1,4-butanediol diglycidyl ether having a proportion of 40 percent by mass to the mass of the gel product was added and stirred for 4 hours. The system was added with 500 microliters of aqueous solution containing 40 wt% sodium hydroxide and 5 wt% sodium borohydride, and the mixture was stirred for 12 hours. The system was added with epichlorohydrin having a proportion of 50 percent by mass to the mass of the reaction mixture, and 1500 microliters of an aqueous solution containing 40 wt% sodium hydroxide were mixed and added to the reaction mixture, and the mixture was stirred for 12 hours. The resulting polymeric microparticles were washed until pH neutral, and then were soaked in solutions of HCl until no bubbles were generated. After sieving, the microparticles that have the particle size between 40µm and 150µm are retained. The optical conformation of the particles before and after soaking under a polarizing microscope was observed and a freeze dryer was adopted for shaping, and then the morphology and aperture characteristics are observed by scanning electron microscopy (SEM). As shown in FIG.17, the pore diameter of the macropores identified as 4.37um. Since the internal structure of the microparticles is obviously destroyed, resulting in the collapse of the microparticles' surface.

In summary, it can be seen from the above-mentioned embodiments and comparative example results that the size of the macropores becomes larger as the amount of pore-former increases. No significant structure change was observed in the microspheres with ordered pore structure after the acid treatment, while significant collapse was observed in the microspheres with disordered pore structure. Thus, it can be known that the polymeric microparticles prepared in the present application which include both macropores and ordered internal gel pores, can improve the permeability of the separation matrice, which tends to generate a convective flow within the matrice. Therefore, it is suitable for a relatively higher flow rate and thereby results in improving the separation efficiency.

It should be understood that although the examples described above provided certain specific embodiments in accordance with the present invention, those embodiments are exemplary, that such a description manner is only for the sake of clarity, that those skilled in the art should take the description as an integral part, and that the technical solutions in the embodiments may be suitably combined to form other embodiments understandable by those skilled in the art.

The detailed descriptions set forth above are merely specific illustrations of feasible embodiments of the present invention, and are not intended to limit the scope of protection of the present invention. All equivalent embodiments or modifications that do not depart from the art spirit of the present invention should fall within the scope of protection of the present invention.

## Claims

1. Polymeric microparticle with dual size pores, wherein the polymeric microparticles are formed by crosslinking at least partially cross-linkable oligomer materials, including rigid nanoparticles, wherein at least one of the rigid nanoparticles has a non-spherical shape in a solution. The polymeric microparticles have two sets of pores having distinctive sizes distributed inside, wherein the first set of pores are macropores larger in size, and the second set of pores are gel pores that are smaller in size and formed internal structural ordering at least in regions, wherein the pore diameter of the macropores range from 2-20 µm, the pore diameter of the gel pores range from 1-1000 nm.

2. The polymeric microparticles according to claim 1, wherein the rigid nanoparticle is a biomacromolecule.

3. The polymeric microparticles according to claim 1, wherein the shape of the non-spherical rigid nanoparticles is rod-shaped, strip-shaped, sheet-shaped, needle-shaped, or linear with its feature direction along the direction of the longitudinal axis of the molecule.

4. The polymeric microparticles according to claim 1, wherein the shape of the non-spherical rigid nanoparticles is disk-shaped with its feature direction normal to the disk.

5. The polymeric microparticles according to claim 1, wherein at least in a substantially ordered area, at least a portion of the gel pore and at least a portion of its adjacent one has a certain correlation with the arrangement of their directions and positions.

6. The polymeric microparticles according to claim 5, wherein at least in a substantially ordered area, at least a portion of the gel pore is substantially parallel, fan-shaped, or spirally arranged with at least a portion of its adjacent one.

7. The polymeric microparticles according to claim 1, wherein the polymeric microparticles further comprise a polysaccharide compound having no obvious non-spherical shape, and the polysaccharide compound and the rigid nanoparticles are copolymerized to form the polymeric microparticles.

8. The use of the polymeric microparticles according to any one of claims 1 to 7 as stationary phases for chromatographic separations.

9. A method of preparing the polymeric microparticles, the method comprising: mixing and emulsifying an aqueous phase containing the rigid nanoparticles and the pore-former and an oil phase which are mutually insoluble with the aqueous phase, after curing to crosslink, the pore-former is then removed to give the polymeric microparticles containing both the ultra large pores and ordered pores.

10. The method according to claim 9, wherein the method specifically comprises the steps of:
a) dispersing the rigid nanoparticles and the pore-formers into water to form a dispersed phase solution;
b) dispersing the dispersed phase solution in a continuous phase containing an emulsifier to form emulsion droplets containing the rigid nanoparticles;
c) adding a cross-linking agent to the thus-obtained product to cross-link the rigid nanoparticles in the emulsion droplets to form the polymeric microparticles after the continuous phase solvent is removed;
d) removing the pore-former by washing, to obtain the polymeric microparticles with dual size pores.

11. The method according to claim 10, wherein the rigid nanoparticle is a biomacromolecule.

12. The method according to claim 10, wherein step a) hereinbefore described further comprises the step of adding a polysaccharide compound.

13. The method according to any one of claims 9 or 10, wherein the pore-former is selected from inorganic salts, single-stranded RNA viruses or metallic oxides.

14. The method according to claim 13, wherein at least one member of the pore-former is selected from the group consisting of magnesium carbonate, barium carbonate, calcium carbonate, aluminium oxide, and tobacco mosaic virus.

15. The method according to claim 10, wherein the emulsifier is selected from nonionic surfactant or anionic surfactant.
